(19) Europäisches Patentamt
European Patent Office
Office européen des brevets

(11) Numéro de publication: **0 212 309 A2**

(12) # DEMANDE DE BREVET EUROPEEN

(21) Numéro de dépôt: 86110138.4

(51) Int. Cl.⁴: C07H 3/08

(22) Date de dépôt: 23.07.86

(30) Priorité: 19.08.85 CH 3562/85

(43) Date de publication de la demande: 04.03.87 Bulletin 87/10

(84) Etats contractants désignés: CH DE FR GB LI NL

(71) Demandeur: FIRMENICH SA
1, route des Jeunes
CH-1211 Genève 8(CH)

(72) Inventeur: Delay, François, Dr.
9, rue des Pervenches
CH-1227 Carouge(CH)
Inventeur: Mazenod, François, Dr.
16, Cité Vieusseux
CH-1203 Geneve(CH)
Inventeur: Naef, Ferdinand, Dr.
30, chemin Vert
CH-1227 Carouge(CH)

(74) Mandataire: Salvadori, Giuseppe, Dr.
c/o Firmenich S.A. Case Postale 239
CH-1211 Genève 8(CH)

(54) ## Procédé pour la préparation de 6-desoxy-D-glucose.

(57) Le 6-désoxy-D-glucose, intermédiaire utile pour la préparation de Furanéol (marque enregistrée de Firmenich SA, Genève), est préparé par halogénation de méthyl alpha-D-glucopyranoside au moyen de phosgène en présence de N,N-diméthylformamide et hydrogénolyse du dérivé chloré résultant.

Selon une variante dudit procédé, le dérivé chloré est converti en dérivé iodé ou bromé correspondant avant son hydrogénolyse. Ses dérivés iodé, respectivement bromé, peuvent être obtenus en tant qu'intermédiaires de transition suivant une réaction de réduction cathodique du composé chloré en présence d'iodure de sodium dans le N,N-diméthylformamide.

Le 6-désoxy-D-glucose désiré est enfin obtenu par hydrolyse du produit résultant en milieu acide.

EP 0 212 309 A2

## Procédé pour la préparation de 6-désoxy-D-glucose

Le 6-désoxy-D-glucose, également connu sous la dénomination de quinovose, épifucose ou encore de D-isorhamnose, est un sucre d'origine naturelle extrait de l'écorce de quinquina. Il peut être préparé par réduction de 6-O-p-toluènesulfonyl-D-glucose à l'aide d'aluminohydrure de lithium selon la méthode décrite par O. Th. Schmidt dans Methods in Carbohydrate Chemistry I, Academic Press, p. 198 (1962). Il peut également être préparé par réduction avec le $LiAlH_4$ du méthyl 6-chloro-6-désoxy-alpha-D-glucopyranoside conformément à la méthode décrite par M. E. Evans et F. W. Parrish dans Methods in Carbohydrate Chemistry VI, Academic Press, p. 177 (1972) suivant le schéma réactionnel (A).

Nous avons découvert que deux des étapes du procédé illustré ci-dessus pouvaient être sensiblement améliorées et cette observation est à la base de notre invention.

La présente invention a en effet pour objet un procédé pour la préparation de 6-désoxy-D-glucose, lequel procédé est caracteérisé en ce que l'halogénation de méthyl alpha-D-glucopyranoside s'effectue à l'aide de phosgène dans le N,N-diméthylformamide (DMF) et la réduction du méthyl 6-chloro-6-désoxy-alpha-D-glucopyranoside obtenu s'opère par l'action d'une hydrogénation catalytique en présence de nickel de Raney.

Selon une variante du procédé de l'invention, le méthyl 6-chloro-6-désoxy-alpha-D-glucopyranoside obtenu est converti en ses dérivés iodé ou bromé par réaction avec l'iodure, respectivement le bromure de sodium dans un solvant cétonique ou dans le DMF. Cette étape est suivie de l'hydrogénolyse, comme indiqué plus haut, par hydrogénation catalytique des composés obtenus en présence de nickel de Raney.

Les deux étapes successives d'échange du chlore, par l'iode ou le brome, et d'hydrogénolyse peuvent également s'effecteur avantageusement en une seule opération. Dans ce cas, l'on peut opérer avec des quantités d'iodure ou bromure de sodium inférieures à la quantité équivalente requise, ce qui rend le procédé plus économique. On peut ainsi utiliser des quantités comprises entre environ 0,05, voire inférieures, et 1,00 équivalent de NaI pour un équivalent de méthyl 6-chloro-6-désoxy-alpha-D-glucopyranoside de départ, l'iodure requis par la réaction étant régénéré au fur et à mesure de sa consommation au cours de l'hydrogénolyse.

La réaction s'effectue en présence d'une base afin de neutraliser la quantité d'acide formé. A cet effet, on peut utiliser un hydroxyde de métal alcalin, tel l'hydroxyde de sodium ou potassium, ou un carbonate, tel le carbonate de sodium ou potassium. La réaction s'effectue également à une pression supérieure à la pression atmosphérique. Selon un mode d'exécution particulier, on opère à une pression comprise entre environ 5 et 200 bars et à une température comprise entre environ 50° et 200°C.

Comme il a été indiqué ci-dessus, la réaction s'effectue dans un solvant cétonique ou dans le DMF. A titre de solvant cétonique, il convient d'utiliser une cétone dialkylée, l'acétone ou la diéthylcétone, ou une cétone cycloaliphatique telle par exemple la cyclohexanone ou la dihdydroisophorone, ou encore une lactame, telle la N-méthylpyrrolidone.

Suivant un mode d'exécution particulier de la présente invention, on soumet à hydrogénolyse le méthyl 6-chloro-6-désoxy-alpha-D-glucopyranoside en solution dans le DMF à une concentration d'environ 10% en poids en présence d'environ 0,5 équivalents d'iodure de sodium pour 1 équivalent de glucopyranoside de départ, et d'environ 1 équivalent de KOH.

La réaction est conduite à une pression d'environ 5 bars et à 150°C. Dans ces conditions, le temps de réaction est de 11 heures environ et le rendement observé en méthyl 6-désoxy-alpha-D-glucopyranoside était de 87,3%. La conversion de méthy 6-chloro-6-désoxy-alpha-D-glucopyranoside en dérivé iodé correspondant, méthyl 6-iodo-6-désoxy-alpha-D-glucopyranoside, avant hydrogénolyse, conduit à une réduction substantielle des valeurs de pression et température par comparaison à celles nécessaires pour effecteur l'hydrogénolyse dudit composé chloré. C'est ainsi que l'hydrogénolyse en présence de nickel de Raney de méthyl 6-iodo-6-désoxy-alpha-D-glucopyranoside isolé conduit, à 40°C et 1 bar de pression d'hydrogène, au méthyl 6-désoxy-alpha-D-glucopyranoside désiré en 16 heures, avec un rendement d'environ 96%.

Suivant une autre variante du procédé de l'invention, on obtient le méthyl 6-désoxy-alpha-D-glucopyranoside en soumettant le méthyl 6-chloro-6-désoxy-alpha-D-glucopyranoside à une réduction cathodique en présence d'iodure de sodium dans le DMF. A cet effet, on opère de préférence avec une cathode de mercure dans une cellule à compartiments anodique et cathodique séparés par une membrane échangeuse de cation [par exemple : Nafion 117 (Nafion est une marque enregistrée)].

L'apport des protons nécessaires à la réduction est assuré par la réaction d'oxydation anodique d'hydrate d'hydrazine qui se transforme ainsi en azote avec libération de 4 équivalents de protons sur une anode de platine.

Le pH du compartiment cathodique reste done constant et se situe au voisinage de 7,5.

Comme il a été indiqué plus haut, la première étape du procédé de l'invention est caractérisée par une halogénation du méthyl alpha-D-glucopyranoside au moyen de phosgène dans le DMF. Le méthyl 6-chloro-6-désoxy-alpha-D-glucopyranoside est ainsi obtenu avec un excellent rendement - (presque quantitatif) suivant un procédé simple d'application industrielle aisée. Une telle méthode s'est révélée être plus avantageuse que celle qui avait été décrite par M. E. Evans et F. W. Parrish - [Methods in Carbohydrate Chemistry, VI, Academic Press, p. 193 (1972)]. En effet, bien que les rendements observés soient comparables à ceux obtenus par la méthode citée, la réaction procède sans formation de sels pouvant gêner la purification suivante du produit. Avec le phosgène, la réaction procède en effet avec élimination d'acide chlorhydrique et d'anhydride carbonique. D'autre part, la chloruration à l'aide du chlorure de méthanesulfonyle, telle que décrite par Evans et Parrish, procède en utilisant un large excès de ce réactif, tandis qu'il suffit d'utiliser un léger excès de phosgène pour effectuer une conversion complète de méthyl alpha-D-glucopyranoside.

Le phosgène peut être employé soit en phase gazeuse, soit en solution dans un solvant organique inerte approprié, par exemple dans un hydrocarbure aromatique, tel le toluène.

Le procédé de l'invention peut être illustré à l'aide du schéma réactionnel (B).

Le méthyl alpha-D-glucopyranoside, utilisé comme produit de départ pour le procédé de l'invention, est un produit commercial ou qui peut être préparé conformément aux méthodes décrites, par exemple par G. N. Bollenback [Methods in Carbonhydrate Chemistry II, p. 326 (1963)].

Le 6-désoxy-D-glucose obtenu est un produit intermédiaire utile pour la préparation de FURANEOL (marque enregistrée de Firmenich SA, Genève) ou 2,5-diméthyl-4-hydroxy-2,3-dihydrofuran-3-one (voir demande de brevet japonais 79 19962).

L'invention est illustrée de manière plus détaillée par les exemples suivants dans lesquels les températures sont indiquées en degrés centigrades et les abréviations ont le sens usuel dans l'art.

Exemple 1

Préparation de méthyl 6-chloro-6-désoxy-alpha-D-glucopyranoside

120 g (0,62 M) de méthyl alpha-D-glucopyranoside ont été dissous dans 2300 ml de DMF anhydre et la solution a été placée sous azote dans un réacteur de 4 lt muni d'un réfrigérant, d'un thermomètre, d'une ampoule à addition et d'une agitation mécanique. A la solution obtenue, on a ensuite ajouté goutte à goutte 490 ml d'une solution à 20% de phosgène dans le toluène (1,5 équivalents) en maintenant la température de la solution entre -10° et 0°. La solution incolore résultante a été ensuite agitée 6 h à température ambiante, puis 6 h à 80°. Les parties volatiles ont été ensuite distillées sous pression réduite (10 mmHg) et l'on a obtenu un résidu visqueux brun qui cristallise partiellement. Ce résidu a été dissous dans un mélange acétate d'éthyle-éthanol et eau - (45:5:3) et l'acidité restante a été neutralisée avec une solution aqueuse concentrée de NaOH et la solution a été éluée rapidement sur une colonne de 600 g de silice. On a ainsi obtenu 111,5 g (0,525 M ; rend. 85%) de méthyl 6-chloro-6-désoxy-alpha-D-glucopyranoside sous forme d'un solide jaunâtre. Le produit peut être recristallisé dans l'acétate d'éthyle F. 112-113°.

Exemple 2

Préparation de méthyl 6-chloro-6-désoxy-alpha-D-glucopyranoside

10 g (51,5 mM) de méthyl alpha-D-glucopyranoside ont été dissous dans 200 ml de DMF anhydre et la solution a été placée dans un réacteur de 350 ml muni d'un réfrigérant, d'un thermomètre, d'une agitation magnétique et d'un tube d'introduction de gaz. Ce dernier a été relié à un cylindre de phosgène liquéfié par l'intermédiaire d'un dispositif de condensation du gaz, ainsi que des flacons de sécurité habituels en pareille circonstance. 5.5 ml (1,5 équiv.) de phosgène ont alors été condensés dans le dispositif de condensation, puis évaporés lentement et entraînés à travers la solution de méthyl alpha-D-glucopyranoside dans le DMF refroidie à -5°C à l'aide d'un faible courant d'azote. A la fin de l'évaporation du phosgène et de la purge de tout le système par l'azote, la solution a été agitée 3 h à température ambiante, puis 4 h à 80°C. Le traitement du mélange réactionnel a été fait comme dans l'exemple 1. On

a obtenu 10,7 g .de méthyl 6-chloro-6-désoxy-alpha-D-glucopyranoside, soit un rendement de 98%.

Exemple 3

Préparation de méthyl 6-désoxy-alpha-D-glucopyranoside

a. par hydrogénolyse de méthyl 6-chloro-6-désoxy-alpha-D-glucopyranoside

L'hydrogénolyse a été effectuée sur le pyranoside en solution aqueuse ou méthanolique dans un autoclave à une pression d'hydrogène de 200 bars et à 60° en présence de nickel de Raney. La réaction a été conduite en présence d'un équivalent de KOH. Dans ces conditions de réaction, la durée de conversion est relativement longue, de l'ordre de 45 à 92 heures. Toutefois, les rendements observés sont élevés (89,4-93,2%).

b. par hydrogénolyse de méthyl 6-iodo-ou méthyl 6-bromo-6-désoxy-alpha-D-glucopyranoside

1 Equivalent de méthyl 6-chloro-6-désoxy-alpha-D-glucopyranoside a été transformé quantitativement en son dérivé iodé et bromé correspondant par réaction avec un équivalent d'iodure, respectivement bromure de sodium dans la diéthylcétone à 100°.

En effectuant l'hydrogénolyse, non pas sur le dérivé chloré, comme indiqué plus haut, maid iodé (ou bromé), on a obtenu le glucopyranoside désiré en appliquant des conditions de réaction plus douce (pression : 1 bar ; temp. : 40° ; temps de réaction : 16 h). Les rendements observés étaient de 95-98%.

Le tableau (I) résume les résultats obtenus ainsi ques les conditions de réaction appliquées.

c. par hydrogénolyse de méthyl 6-chloro-6-désoxy-alpha-D-glucopyranoside en présence de NaI

En effectuant l'hydrogénolyse sur le dérivé chloré, mais en présence de NaI, on a pu observer une diminution sensible des temps de réaction. Le tableau (II) résume les résultats obtenus ainsi que les conditions de réaction appliquées. La réaction a été effectuée dans le DMF en présence d'un équivalent de $Na_2CO_3$ et de 10% en poids de nickel de Raney.

La procédure générale type suivie a été la suivante.

On a chargé un autoclave avec 1 g (4,7 mM) de méthyl 6-chloro-6-désoxy-alpha-D-glucopyranoside, 0,5 g (4,7 mM) de carbonate de sodium, 0,47 mM de NaI, 10 ml de DMF et environ 0,1 g de nickel de Raney, préalablement rincé avec du méthanol et du DMF. Le mélange a été purgé avec de l'argon et l'autoclave fermé ; ensuite, après avoir purgé avec de l'hydrogène, on a pressurisé à la pression requise avec l'hydrogène. Le mélange a été chauffé à la température choisie et maintenu sous agitation par l'emploi d'un agitateur magnétique.

Le déroulement de la réaction peut être suivi par analyse HPLC. Une fois la réaction terminée, le mélange a été filtré et, sous pression réduite, on a évaporé le DMF, qui peut être ainsi recyclé.

Le produit brut a été dissous dans l'eau et les sels formés (NaCl et NaI) éliminés par filtration sur une résine du type Biorad AG 501 (résine mixte).

Par évaporation de l'eau, on a obtenu un produit visqueux, sirupeux brun clair qui cristallise lentement.

Le tableau (III) résume les résultats obtenus lors de l'hydrogénolyse en présence de NaBr à la place de NaI.

Exemple 4

Préparation de méthyl 6-désoxy-alpha-D-glucopyranoside par réduction cathodique

Une solution constituée par 7,5 g de méthyl 6-chloro-6-désoxy-alpha-D-glucopyranoside et 10 g de NaI dans 350 ml de DMF e été circulée d'abord dans un réacteur chauffé à 100-150°; puis à travers un échangeur de chaleur afin d'en abaisser la température à environ 50°, puis à travers le compartiment cathodique d'une cellule d'électrolyse avant d'être recylée dans le réacteur. L'opération est effectuée jusqu'à conversion totale du produit de départ. La cellule d'électrolyse utilisée comportait une cathode en mercure et elle était du type à compartiments séparés. Elle comportait à cet effet une membrane échangeuse de cation Nafion 117 - (Nafion est une marque enregistrée). Le pH du compartiment cathodique est resté à une valeur constante autour de 7,5 pendant tout le déroulement de la réaction.

Une fois la réaction terminée, le DMF a été

évaporé, le résidu a été repris à l'acétone et les sels inorganiques formés on été éliminés par filtration. Le méthyl 6-désoxy-alpha-D-glucopyranoside a été enfin obtenu par évaporation du filtrat clair cétonique et purifié par chromatographie sur une colonne remplie de silice.

**ANNEXE** 1/2

SCHEMA (A)

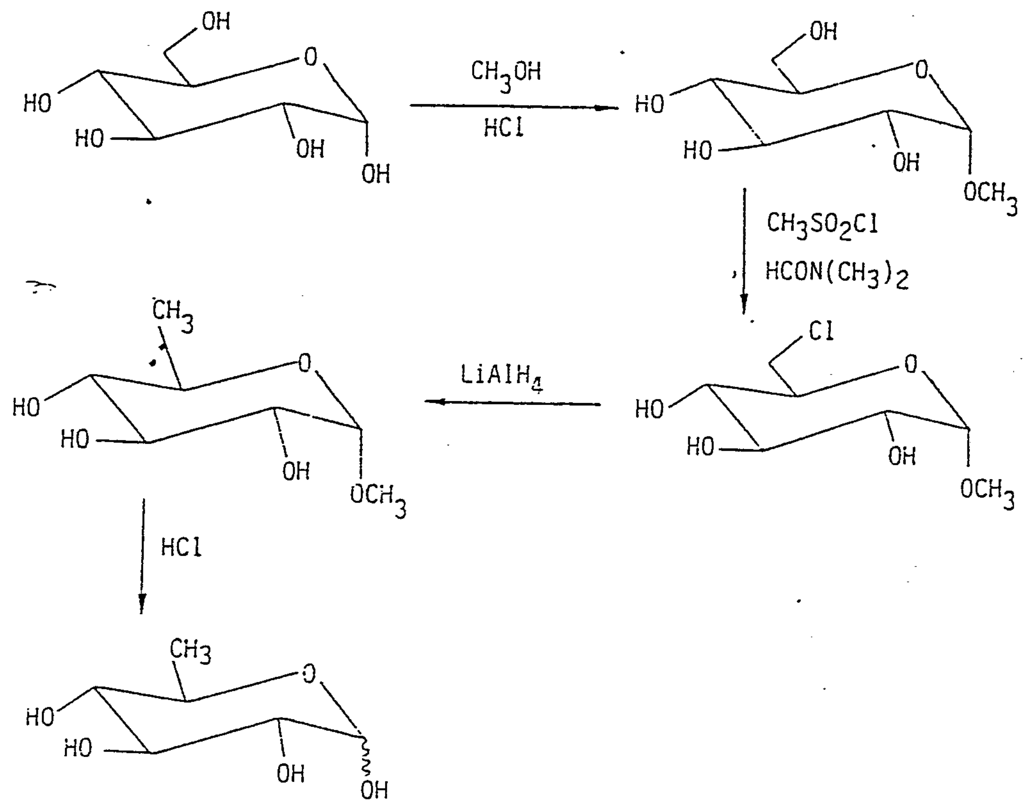


SCHEMA (B)

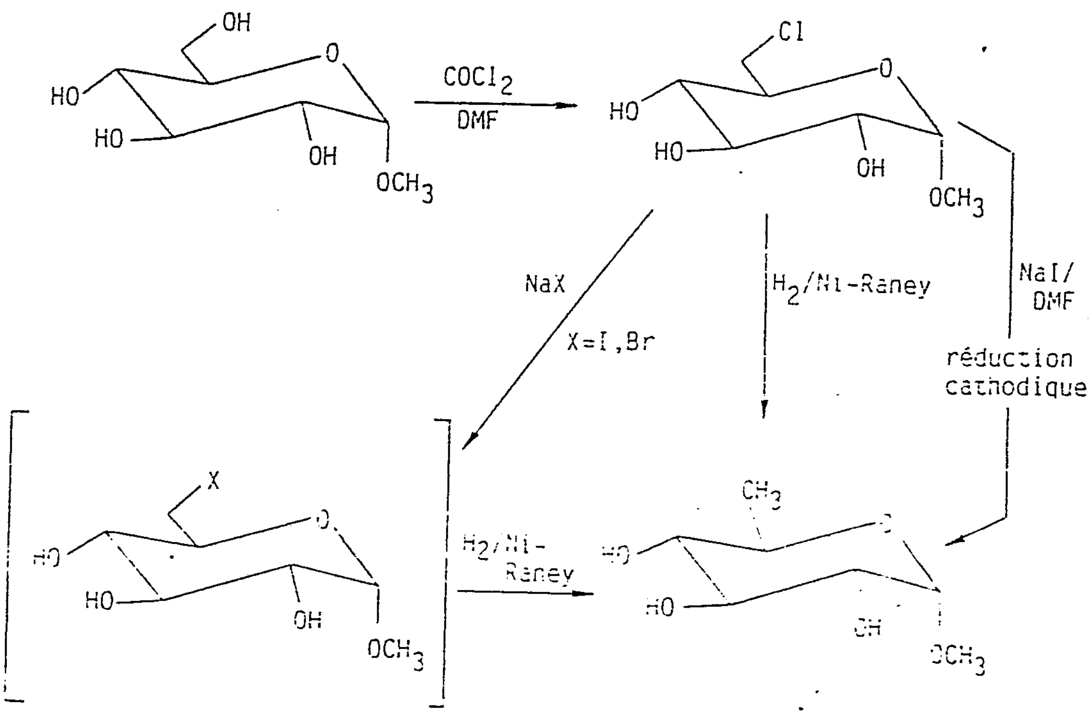

ANNEXE 2/2

TABLEAU (I)

| | Conc. de 1) [% en poids] | Solvant | Base | Catalyseur | Pression [bar] | Température [°C] | Temps [h] | Rend. théor. [%] |
|---|---|---|---|---|---|---|---|---|
| 1 | 2 | MeOH | 1 éq. $Na_2CO_3$ | 10% Ra-Ni | 1 | 40 | 15 | 96 |
| 2 | 2,7 | $MeOH/H_2O$ | 1 éq. $NaHCO_3$ | 9,3% Ra-Ni | 5 | 60 | 3 | 82 |
| 3 | 3,5 | $Et_2CO$ | ← | 7,1% Ra-Ni | 5 | 100 | 23 | 0 |
| 4 | 3,5 | $Et_2CO$ | 1 éq. $Et_3N$ | 7,1% Ra-Ni | 5 | 40 | 48 | 84 |

1) méthyl 6-chloro-6-désoxy-alpha-D-glucopyranoside

TABLEAU (II)

| | Conc. de 1) [% en poids] | NaI [M équivalents] | Température [°C] | Pression [bar] | Temps [h] | Rend. théor. [%] |
|---|---|---|---|---|---|---|
| 1 | 2,5 | 1 | 150 | 5 | 4,75 | 80,0 |
| 2 | 2,5 | 0,5 | 150 | 5 | 10 | 75 |
| 3 | 10 | 0,5 | 150 | 5 | 11 | 87,3 |
| 4 | 10 | 0,2 | 150 | 5 | 5 | 71,0 |
| 5 | 10 | 0,6 | 200 | 10 | 2,5 | 45,0 |
| 6 | 20 | 0,05 | 150 | 5 | 16 | 51,0 |
| 7 | 20 | 0,05 | 150 | 5 | 24 | 27,0 |

1) méthyl 6-chloro-6-désoxy-alpha-D-glucopyranoside

TABLEAU (III)

| | Conc. de 1) [% en poids] | NaI [M équivalents] | Température [°C] | Pression [bar] | Temps [h] | Rend. théor. [%] |
|---|---|---|---|---|---|---|
| 1 | 2,5 | 10 | 100 | 5 | 47 | 37 |
| 2 | 2,5 | 1 | 100 | 5 | [illegible] | 43,6 |
| 3 | 10 | 0,1 | 150 | 5 | 14 | 39,4 |

1) méthyl 6-chloro-6-désoxy-alpha-D-glucopyranoside

## Revendications

1. Procédé pour la préparation de 6-désoxy-D-glucose par halogénation de méthyl alpha-D-glucopyranoside suivie de réduction et hydrolyse, caractérisé en ce que

a. on effectue ladite halogénation au moyen de phosgène dans une solution de N,N-diméthylformamide,

b. on hydrogénolyse ensuite, en présence d'une base, le méthyl 6-chloro-6-désoxy-alpha-D-glucopyranoside obtenu par hydrogénation catalytique en présence de nickel de Raney, ou

c. on convertit le méthyl 6-chloro-6-désoxy-alpha-D-glucopyranoside en méthyl 6-iodo-ou méthyl 6-bromo-6-désoxy-alpha-D-glucopyranoside par réaction avec l'iodure, respectivement le bromure de sodium dans un solvant cétonique ou dans le N,N-diméthylformamide et hydrogénolyse le composé obtenu comme indiqué sous lettre b. ci-dessus, ou

d. on hydrogénolyse, en présence d'une base et d'iodure ou bromure de sodium, le méthyl 6-chloro-6-désoxy-alpha-D-glucopyranoside dans un solvant cétonique ou dans le N,N-diméthylformamide, ou

e. on soumet le méthyl 6-chloro-6-désoxy-alpha-D-glucopyranoside à une réduction cathodique en présence d'iodure de sodium dans le N,N-diméthylformamide pour fournir le méthyl 6-désoxy-alpha-D-glucopyranoside, et

f. on hydrolyse enfin ledit composé en milieu acide.

2. Procédé selon la revendication 1, caractérisé en ce que l'hydrogénolyse s'effectue en présence d'un hydroxyde ou d'un carbonate de métal alcalin en tant que base, à un pression supérieure à la pression atmosphérique et à une température comprise entre 50° et 200°C.

3. Procédé selon la revendication 1, caractérisé en ce que, lors de l'étape d., le méthyl 6-chloro-6-désoxy-alpha-D-glucopyranoside est soumis à hydrogénolyse en solution dans un solvant cétonique ou dans le N,N-diméthylformamide à une concentration comprise entre environ 2,5 et 20% en poids.

4. Procédé selon la revendication 1, caractérisé en ce que, lors de l'étape d., l'iodure de sodium est présent à une concentration inférieure à la quantité stoéchiométrique.

5. Procédé selon les revendications 2 à 4, caractérisé en ce que la concentratin d'iodure de sodium est comprise entre environ 0,05 et 1,00 équivalent pour un équivalent de méthyl 6-chloro-6-désoxy-alpha-D-glucopyranoside de départ.

6. Procédé selon la revendication 5, caractérisé en ce qu'on soumet à hydrogénolyse le méthyl 6-chloro-6-désoxy-alpha-D-glucopyranoside en solution dans le N,N-diméthylformamide à une concentration d'environ 10% en poids en présence d'environ 0,5 équivalents d'iodure de sodium pour 1 équivalent de glucopyranoside de départ et d'environ 1,0 équivalent de KOH, et qu'on opère à une pression d'environ 5 bars et à une température d'environ 150°C.

7. Procédé selon la revendication 1, caractérisé en ce que, lors de l'étape e., l'on opère à l'aide d'une cathode de mercure dans une cellule à compartiments cathodique et anodique séparés.

8. Procédé selon la revendication 7, caractérisé en ce que la réduction cathodique s'effectue en continu.

9. Procédé selon la revendication 7, caractérisé en ce que le méthyl 6-désoxy-alpha-D-glucopyranoside résultant est isolé par évaporation de l'excès de N,N-diméthylformamide présent, traitement du résidu obtenu avec de l'acétone, filtration des sels inorganiques formés, concentration de la solution organique claire et chromatographie sur une colonne remplie de silice.